Europäisches Patentamt

European Patent Office

Office européen des brevets

(19)

(11) Veröffentlichungsnummer : **0 331 623 B1**

# (12) EUROPÄISCHE PATENTSCHRIFT

(45) Veröffentlichungstag der Patentschrift :
21.08.91 Patentblatt 91/34

(51) Int. Cl.⁵ : **A61F 2/36**

(21) Anmeldenummer : **89810030.0**

(22) Anmeldetag : **12.01.89**

(54) Gerader Verankerungsschaft für eine Femurkopfprothese.

(30) Priorität : 29.02.88 CH 746/88

(43) Veröffentlichungstag der Anmeldung :
06.09.89 Patentblatt 89/36

(45) Bekanntmachung des Hinweises auf die
Patenterteilung :
21.08.91 Patentblatt 91/34

(84) Benannte Vertragsstaaten :
AT BE DE ES FR GB IT NL SE

(56) Entgegenhaltungen :
EP-A- 0 058 744
EP-A- 0 143 847
WO-A-86/02260
US-A- 4 561 432

(73) Patentinhaber : GEBRÜDER SULZER
AKTIENGESELLSCHAFT
Zürcherstrasse 9
CH-8401 Winterthur (CH)

(72) Erfinder : Willert, Hans-Georg, Prof. Dr.-med.
Schlegelweg 9
W-3400 Göttingen (DE)
Erfinder : Semlitsch, Manfred, Dr.
Endlikerstrasse 82
CH-8400 Winterthur (CH)

## Beschreibung

Die Erfindung betrifft einen geraden Verankerungsschaft für eine Femurkopfprothese, der mit Hilfe eines Knochenzementbettes im Oberschenkel fixiert werden soll, wobei in der zentralen Längsachse des Schaftes eine Bohrung verläuft, die den ganzen Schaft durchsetzt und einen in sie einschiebbaren Zentrierstab aufnimmt, an dessen Dicke der Durchmesser der Bohrung angepasst ist.

Für eine gute Haftung des Schaftes in einem Zementbett einerseits und des Zementbettes im umgebenden Knochengewebe andererseits ist es erforderlich, dass der den Schaft umhüllende Knochenzement überall eine Mindestdicke von beispielsweise 1-3 mm hat und darüberhinaus zumindest über den Umfang möglichst gleichmässig verteilt ist.

Aus der WO-86/02260 ist ein Geradschaft der eingangs genannten Art bekannt. Bei dieser Konstruktion ist der mit seinem Kopf im proximalen Ende des Schaftes versenkte Zentrierstab am distalen Ende mit einem ringförmigen Element verschraubt, das durch Drehen des Stabes so verformt wird, dass eine Anzahl dornenartiger Vorsprünge zur zentralen Fixierung des distalen Schaftendes in das Knochengewebe hineingepresst wird. Das Element bewirkt so nicht nur diese distale Zentrierung des Schaftes, sondern dient gleichzeitig als Zementsperre.

Unabhängig davon, ob der Schaft vor oder nach dem Knochenzement in den Knochen eingebracht wird, ist es für den Operateur schwierig, den zwar am distalen Ende zentrierten Schaft auf seiner ganzen Länge zentriert zu halten bzw. zentriert in den Knochenzement einzupressen.

Aufgabe der Erfindung ist es, dem Operateur die Arbeit beim Einsetzen und Einzementieren des Schaftes in einer zentralen Lage zu erleichtern.

Diese Aufgabe wird dadurch gelöst, dass die Länge des Zentrierstabes mindestens die doppelte, in der Längsachse gemessene Länge des Schaftes beträgt.

Bei der Implantation wird der Zentrier- oder Führungsstab zunächst in der fiktiven Mittelachse des Operationshohlraumes angeordnet und gehalten; anschliessend wird der Knochenzement in den Hohlraum eingefüllt. Vorteilhafterweise kann der Zentrierstab dabei lösbar in einer Markraumsperre, die in bekannter Weise das Zementbett gegen das Knocheninnere abschliesst, befestigbar sein.

Die Länge des Zentrierstabes entspricht mindestens der doppelten Schaftlänge, so dass der Schaft leicht aufgefädelt und geführt in den weichen Knochenzement hineingedrückt werden kann ; er wird dadurch besonders in den Richtungen anterior/posterior und medial/lateral zentriert. Der Zentrierstab wird nun mit seinem herausragenden Ende eingespannt oder an diesem möglichst unbeweglich gehalten, bis der Knochenzement soweit ausgehärtet ist, dass der Schaft lagestabil gehalten wird.

Anschliessend kann der Zentrierstab entfernt.

Im folgenden wird die Erfindung anhand eines Ausführungsbeispiels näher erläutert.

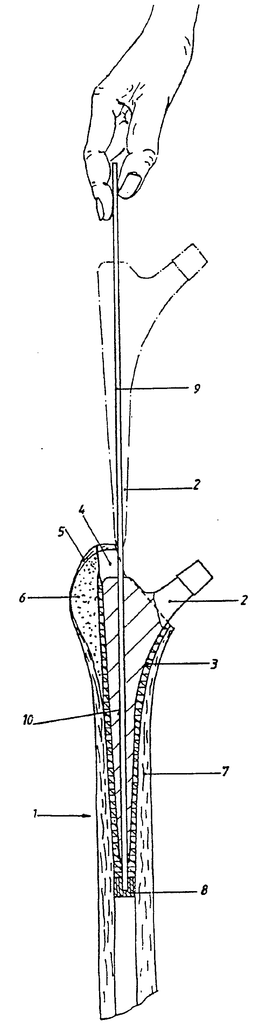

Die einzige Figur zeigt in einem Längsschnitt durch einen Femur eine "Momentaufnahme" bei der Implantation, wobei der Zeitpunkt festgehalten ist, zudem der Schaft in ein Knochenzementbett eingepresst ist.

In dem durch einen Schnitt etwa senkrecht zum Schenkelhals eröffneten Femur 1 ist für die Aufnahme eines Verankerungsschaftes 2 und eines Knochenzementbettes 3 ein Operationshohlraum 4 geschaffen. In diesem ist ausser im Bereich des grossen Trochanters 5 das spongiose Gewebe 6 bis zum Rand der Kortikalis 7 ausgeräumt und durch den Knochenzement 3 ersetzt.

Dessen tieferes Eindringen in den Knochen 1 wird verhindert durch eine Markraumsperre 8 ; in diese ist ein Zentrierstab 9 lösbar eingesetzt, der eine Bohrung 10 in der Längsachse des Schaftes 1 durchsetzt und an seinem oberen Ende gehalten wird.

Die Länge des Zentrierstabes 6 ist dabei so gewählt, dass der Schaft 1 — wie strichpunktiert angedeutet — ausserhalb des Operationshohlraumes 4 vollständig auf den Stab 9 aufgefädelt werden kann, bevor er in den Knochenzement 3 hineingedrückt wird.

Nach einer Anfangsaushärtung des Zementes wird der Stab 9 aus der Markraumsperre 8 gelöst und entfernt.

## Patentansprüche

1. Gerader Verankerungsschaft (2) für eine Femurkopfprothese, der mit Hilfe eines Knochenzementbettes (3) im Oberschenkel (1) fixiert werden soll, wobei in der zentralen Längsachse des Schaftes (2) eine Bohrung (10) verläuft, die den ganzen Schaft (2) durchsetzt und einen in sie einschiebbaren Zentrierstab (9) aufnimmt, an dessen Dicke der Durchmesser der Bohrung (10) angepasst ist, **dadurch gekennzeichnet, dass** die Länge des Zentrierstabes (9) mindestens die doppelte, in der Längsachse gemessene Länge des Schaftes (1) beträgt.

2. Verankerungsschaft nach Anspruch 1, **dadurch gekennzeichnet, dass** der Zentrierstab (9) an einem Ende lösbar an einer Markraumsperre (8) befestigbar ist.

## Claims

1. A straight anchoring stem (2) for a femoral head prosthesis for fixing in the thigh (1) by means of a bone cement bed (3), a bore (10) being formed in the central longitudinal axis of the stem (2) and extending entirely through the latter and accommodating a centring rod (9) insertable therein, the diameter of the bore (10) being adapted to the thickness of said rod, characterised in that the length of the centring rod (9) is at least twice the length of the stem (1) measured along the longitudinal axis.

2. A straight anchoring stem according to claim 1, characterised in that the centring rod (9) is releasably securable at one end to a medullary cavity plug (8).

## Revendications

1. Tige d'ancrage (2) droite pour prothèse de tête de fémur, qui doit être fixée dans le fémur (1) par une couche (3) de ciment pour os, dans laquelle un perçage (10), qui s'étend suivant son axe longitudinal central et la traverse sur toute sa longueur, loge une tringle de centrage (9) à introduire dans la tige et à l'épaisseur de laquelle est adapté le diamètre du perçage (10), caractérisée en ce que la longueur de la tringle de centrage (9) est au moins égale au double de la longueur de la tige (1), mesurée dans l'axe longitudinal.

2. Tige d'ancrage selon la revendication 1, caractérisée en ce que la tringle de centrage (9) est fixée, de manière amovible, à une extrémité d'une barrière d'espace médullaire (8).

4